(19) Europäisches Patentamt
European Patent Office
Office européen des brevets

(11) Publication number: **0 232 078**
A2

(12) **EUROPEAN PATENT APPLICATION**

(21) Application number: 87300545.8

(22) Date of filing: 22.01.87

(51) Int. Cl.³: C 07 K 7/08
C 07 K 7/10, A 61 K 37/02

(30) Priority: 31.01.86 US 824406

(43) Date of publication of application:
12.08.87 Bulletin 87/33

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Nutt, Ruth F.
Hill Road
Green Lane Pennsylvania 18052(US)

(72) Inventor: Verber, Daniel F.
290 Batleson Road
Ambler Pennsylvania 19002(US)

(72) Inventor: Brady, Stephen F.
20 waterman Avenue
Philadelphia Pennsylvania 19118(US)

(72) Inventor: Lyle, Terry A.
570 Camp Wawa Road
Lederach Pennsylvania 19450(US)

(74) Representative: Hesketh, Alan, Dr.
European Patent Department Merck & Co., Inc. Terlings Park Eastwick Road
Harlow Essex, CM20 2QR(GB)

(54) **Peptides having ANF activity.**

(57) Novel peptides having potent natriuretic activity are disclosed with the following amino acid sequence:

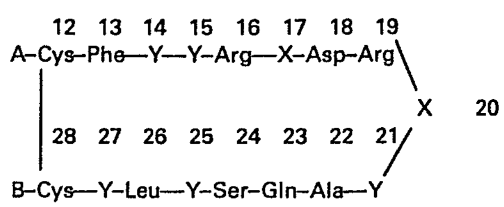

wherein X is L-Ile, D-Ile, D-Ilo-Ile, L-Met or D-Met, Y is Gly, L-Ala or D-Ala, A is H, Ser, Ser-Ser, Arg-Ser-Ser, Arg-Arg-Ser-Ser, Leu-Arg-Arg-Ser-Ser or Ser-Leu-Arg-Arg-Ser-Ser and B is OH, Asn, Asn-Ser, Asn-Ser-Phe, Asn-Ser-Phe-Arg, or Asn-Ser-Phe-Arg-Tyr, and all optically active amino acid residues may be D or L provided at least one amino acid has D-configuration, and the amides, lower alkyl esters and the physiologically acceptable metal salts and acid addition salts thereof.

EP 0 232 078 A2

Croydon Printing Company Ltd.

# PEPTIDES HAVING ANF ACTIVITY

## BACKGROUND OF THE INVENTION

It has been postulated for many years that the cardiac atria serve as sensors that are important in detecting changes in extracellular fluid volume (Gauer *et al.*, Physiol, Rev. 43: 423, 1963). Such a receptor function for the cardiac atria is known in the case of vasopressin, the hypothalmic hormone important in regulating the osmotic concentration of the body fluids.

The postulated existence of a substance which would enhance urinary sodium excretion, and hence be involved in regulation of extracellular fluid volume, was demonstrated recently. de Bold *et al.*, Life Sci. 28: 89, 1981, injected a partially purified extract of cardiac atria of rats into other anesthetized rats and observed a large increase in urine flow and in urinary sodium excretion. This relatively crude extract possessed the appropriate characteristics of an endogenous natriuretic substance.

In addition to its potent diuretic and natriuretic effects, properties that make the material especially appropriate to exert a major effect on body fluid volume regulation, it was also discovered that these extracts of cardiac atria have potent smooth muscle relaxant activity (Currie et al., Science 221: 71, 1983). Such action implies a potential direct role in regulating blood pressure as well as a role in regulating extracellular fluid volume.

Because of the immediately recognized importance of this discovery for understanding the regulation of body fluid volume and blood pressure and the obvious therapeutic potential of such a natural substance in the treatment of congestive heart failure and hypertension, numerous laboratories set about to isolate, characterize and chemically identify the active substance(s) in the cardiac atrial extracts. The active substance(s) in cardiac atria was called atrial natriuretic factor or ANF but has been referred to also as cardionatrin (de Bold et al., Life Sci. 33: 297-302, 1983) and atriopeptin (Currie et al., Science 111: 67, 1984).

DESCRIPTION OF EARLIER ARTICLES AND PATENTS

Thibault et al., FEBS Lett. 164 (2): 286-290 (1983), discloses three peptides of 26, 31 and 33 amino acids and gives their amino acid composition but does not give any amino acid sequences. Since These peptides were isolated from rat atria, all optically active amino acids have L-configuration.

Flynn et al., Biochem. Biophys. Res. Comm. 117 (3): 859-865 (1983), discloses a 28-amino acid

6 7 8 9 10 11

peptide having the sequence Ser-Leu-Arg-Arg-Ser-Ser-

12 13 14 15 16 17 18 19 20 21 22 23 24

Cys-Phe-Gly-Gly-Arg-Ile-Asp-Arg-Ile-Gly-Ala-Gln-Ser-

25 26 27 28 29 30 31 32 33

Gly-Leu-Gly-Cys-Asn-Ser-Phe-Arg-Tyr. Since this peptide was isolated from rat atria, all optically active amino acids have L-configuration.

Currie et al., Science 223: 67-69 (1984), disclose two peptides having sequences 10-30 and 10-32 (numbering as above). Since these peptides were isolated from rat atria, all optically active amino acids have L-configuration.

Kangawa et al., Biochem. Biophys. Res. Comm. 118 (1): 131-139 (1984), disclose a 28-amino acid peptide having sequence 6-33 (numbering as above) having a methionine residue in lieu of isoleucine in 17-position. Since this peptide was isolated from atrial tissue, all optically active amino acids have L-configuration.

Thibault et al., FEBS Lett. 167 (2): 352-357 (1984), disclose isolation of a peptide of 103 amino acids and give the sequence of the C-terminal 73-amino acid fragment. The three peptides disclosed by Thibault et al., supra, correspond to C-terminal fragments of this peptide. Since all of these peptides were isolated from rat atria, and one that was synthesized conformed to the shortest one isolated, all optically active amino acids have L-configuration.

Misono et al., Biochem. Biophys. Res. Comm. 119 (2): 524-529 (1984), disclose isolation of a 25-amino acid peptide of sequence 9-33 (numbering as above). Since this peptide was isolated from rat atria, all optically active amino acids have L-configuration.

Needleman et al., U.S. patent 4,496,544, discloses isolation from several peptides of sequences 12-29, 12-30, 12-32, 12-33, 11-29, 11-30, 11-32, 11-33, 10-29, 10-30, 10-32 and 10-33 (numbering as above). Since all of these peptides were isolated from rat atria, all optically active amino acids have L-configuration.

## OBJECTS OF THE INVENTION

It is an object of the present invention to provide novel peptides having activity like that of ANF peptides isolated from biological materials. Another object is to provide novel peptides having potent natriuretic, vasodilatory and hypotensive activity. A further object is to provide novel peptides having enhanced metabolic stability. These and other objects of the present invention will be apparent from the following description.

## SUMMARY OF THE INVENTION

Novel peptides having potent natriuretic activity are disclosed with the following amino acid sequence:

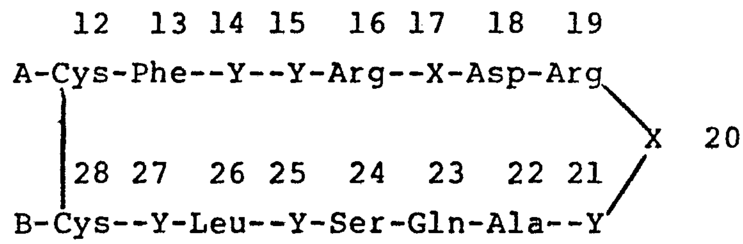

wherein X is L-Ile, D-Ile, D-allo-Ile, L-Met or D-Met, Y is Gly, L-Ala or D-Ala, A is H, Ser, Ser-Ser, Arg-Ser-Ser, Arg-Arg-Ser-Ser, Leu-Arg-Arg-Ser-Ser or Ser-Leu-Arg-Arg-Ser-Ser and B is OH, Asn, Asn-Ser, Asn-Ser-Phe, Asn-Ser-Phe-Arg, or Asn-Ser-Phe-Arg-Tyr, and all optically active amino acid residues may be D or L provided at least one amino acid has D-configuration, and the amides, lower alkyl esters and the physiologically acceptable metal salts and acid addition salts thereof.

DETAILED DESCRIPTION

It has now been found that novel peptides having activity like that of ANF peptides isolated from biological materials, e.g., potent natriuretic, vasodilatory and hypotensive activity, but with enhanced metabolic stability are obtained by substituting at least one D-amino acid for its L-counterpart, or by substituting at least one L-alanine or D-alanine for glycine.

The novel peptides of the present invention are as follows:

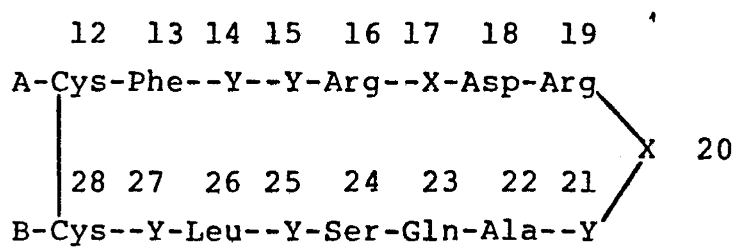

wherein X is L-Ile, D-Ile, D-allo-Ile, L-Met or D-Met, Y is Gly, L-Ala or D-Ala, A is H, Ser, Ser-Ser, Arg-Ser-Ser, Arg-Arg-Ser-Ser, Leu-Arg-Arg-Ser-Ser or Ser-Leu-Arg-Arg-Ser-Ser and B is OH, Asn, Asn-Ser, Asn-Ser-Phe, Asn-Ser-Phe-Arg, or Asn-Ser-Phe-Arg-Tyr, and all optically active amino acid residues may be D or L provided at least one amino acid has D-configuration, and the amides, lower alkyl esters and the physiologically acceptable metal salts and acid addition salts thereof.

D-Amino acids introduce a point at which normal proteolytic enzymes no longer can act. They also modify and rigidify conformation at adjacent amino acids. This is particularly true when glycine is replaced by D-alanine. Decreased conformational flexibility is known to reduce peptide susceptibility to enzyme cleavage. Thus, replacing glycine with L-alanine enhances metabolic stability even though the residue itself is the natural substrate of many proteolytic enzymes. The enhanced potency of some of these analogs probably reflects the decreased flexibility.

Specific peptides of the present invention, including either -COOH or $-CONH_2$ as the C-terminal group, are listed in the following table where the known peptide sequences of L-configuration are shown at the top and the novel peptides of the present invention are obtained by making the substitution indicated. A dash indicates that no amino acid residue is present for the position indicated. The term "DaIle" represents the amino acid D-allo-isoleucine. Conventional abbreviations are used for the other amino acids.

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 1 | – | – | – | – | – | – | D-Cys | " | " | " | " | Ile | " | " | Ile | " | " | " | " | " | " | " | " | – | – | – | – | – |
| 2 | – | – | – | – | – | – | Cys | D-Phe | " | " | " | " | " | " | " | " | " | " | " | " | " | " | " | – | – | – | – | – |
| 3 | – | – | – | – | – | – | " | Phe | D-Ala | " | " | " | " | " | " | " | " | " | " | " | " | " | " | – | – | – | – | – |
| 4 | – | – | – | – | – | – | " | " | L-Ala | " | " | " | " | " | " | " | " | " | " | " | " | " | " | – | – | – | – | – |
| 5 | – | – | – | – | – | – | " | " | Gly | D-Ala | " | " | " | " | " | " | " | " | " | " | " | " | " | – | – | – | – | – |
| 6 | – | – | – | – | – | – | " | " | " | L-Ala | " | " | " | " | " | " | " | " | " | " | " | " | " | – | – | – | – | – |
| 7 | – | – | – | – | – | – | " | " | " | Gly | D-Arg | " | " | " | " | " | " | " | " | " | " | " | " | – | – | – | – | – |
| 8 | – | – | – | – | – | – | " | " | " | " | Arg | D-Ile | " | " | " | " | " | " | " | " | " | " | " | – | – | – | – | – |
| 9 | – | – | – | – | – | – | " | " | " | " | " | D-Met | " | " | " | " | " | " | " | " | " | " | " | – | – | – | – | – |
| 10 | – | – | – | – | – | – | " | " | " | " | " | DaIle | " | " | " | " | " | " | " | " | " | " | " | – | – | – | – | – |
| 11 | – | – | – | – | – | – | " | " | " | " | " | Ile | D-Asp | " | " | " | " | " | " | " | " | " | " | – | – | – | – | – |
| 12 | – | – | – | – | – | – | " | " | " | " | " | " | Asp | D-Arg | " | " | " | " | " | " | " | " | " | – | – | – | – | – |
| 13 | – | – | – | – | – | – | " | " | " | " | " | " | " | Arg | D-Ile | " | " | " | " | " | " | " | " | – | – | – | – | – |
| 14 | – | – | – | – | – | – | " | " | " | " | " | " | " | " | D-Met | " | " | " | " | " | " | " | " | – | – | – | – | – |
| 15 | – | – | – | – | – | – | " | " | " | " | " | " | " | " | DaIle | " | " | " | " | " | " | " | " | – | – | – | – | – |
| 16 | – | – | – | – | – | – | " | " | " | " | " | " | " | " | Ile | D-Ala | " | " | " | " | " | " | " | – | – | – | – | – |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 17 | – | – | – | – | – | – | " | " | " | " | " | " | " | " | " | L-Ala | " | " | " | " | " | " | " | – | – | – | – | – |
| 18 | – | – | – | – | – | – | " | " | " | " | " | " | " | " | " | Gly | D-Ala | " | " | " | " | " | " | – | – | – | – | – |
| 19 | – | – | – | – | – | – | " | " | " | " | " | " | " | " | " | " | Ala | D-Gln | " | " | " | " | " | – | – | – | – | – |
| 20 | – | – | – | – | – | – | " | " | " | " | " | " | " | " | " | " | " | Gln | D-Ser | " | " | " | " | – | – | – | – | – |
| 21 | – | – | – | – | – | – | " | " | " | " | " | " | " | " | " | " | " | " | Ser | D-Ala | " | " | " | – | – | – | – | – |
| 22 | – | – | – | – | – | – | " | " | " | " | " | " | " | " | " | " | " | " | " | L-Ala | " | " | " | – | – | – | – | – |
| 23 | – | – | – | – | – | – | " | " | " | " | " | " | " | " | " | " | " | " | " | Gly | D-Leu | " | " | – | – | – | – | – |
| 24 | – | – | – | – | – | – | " | " | " | " | " | " | " | " | " | " | " | " | " | " | Leu | D-Ala | " | – | – | – | – | – |
| 25 | – | – | – | – | – | – | " | " | " | " | " | " | " | " | " | " | " | " | " | " | " | L-Ala | " | – | – | – | – | – |
| 26 | – | – | – | – | – | – | " | " | " | " | " | " | " | " | " | " | " | " | " | " | " | Gly | D-Cys | – | – | – | – | – |
| 27 | – | – | – | – | – | – | D-Cys | " | " | " | " | " | " | " | " | " | " | " | " | " | " | " | Cys | Asn | – | – | – | – |
| 28 | – | – | – | – | – | – | Cys | D-Phe | " | " | " | " | " | " | " | " | " | " | " | " | " | " | " | " | – | – | – | – |
| 29 | – | – | – | – | – | – | " | Phe | D-Ala | " | " | " | " | " | " | " | " | " | " | " | " | " | " | " | – | – | – | – |
| 30 | – | – | – | – | – | – | " | " | L-Ala | " | " | " | " | " | " | " | " | " | " | " | " | " | " | " | – | – | – | – |
| 31 | – | – | – | – | – | – | " | " | Gly | D-Ala | " | " | " | " | " | " | " | " | " | " | " | " | " | " | – | – | – | – |
| 32 | – | – | – | – | – | – | " | " | " | L-Ala | " | " | " | " | " | " | " | " | " | " | " | " | " | " | – | – | – | – |
| 33 | – | – | – | – | – | – | " | " | " | Gly | D-Arg | " | " | " | " | " | " | " | " | " | " | " | " | " | – | – | – | – |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 34 | - | - | - | - | - | - | " | " | " | " | Arg | D-Ile | " | " | " | " | " | " | " | " | " | " | " | " | - | - | - | - |
| 35 | - | - | - | - | - | - | " | " | " | " | " | D-Met | " | " | " | " | " | " | " | " | " | " | " | " | - | - | - | - |
| 36 | - | - | - | - | - | - | " | " | " | " | " | DaIle | " | " | " | " | " | " | " | " | " | " | " | " | - | - | - | - |
| 37 | - | - | - | - | - | - | " | " | " | " | " | Ile | D-Asp | " | Ile | " | " | " | " | " | " | " | " | " | - | - | - | - |
| 38 | - | - | - | - | - | - | " | " | " | " | " | " | Asp | D-Arg | " | " | " | " | " | " | " | " | " | " | - | - | - | - |
| 39 | - | - | - | - | - | - | " | " | " | " | " | " | " | Arg | D-Ile | " | " | " | " | " | " | " | " | " | - | - | - | - |
| 40 | - | - | - | - | - | - | " | " | " | " | " | " | " | " | D-Met | " | " | " | " | " | " | " | " | " | - | - | - | - |
| 41 | - | - | - | - | - | - | " | " | " | " | " | " | " | " | DaIle | " | " | " | " | " | " | " | " | " | - | - | - | - |
| 42 | - | - | - | - | - | - | " | " | " | " | " | " | " | " | Ile | D-Ala | " | " | " | " | " | " | " | " | - | - | - | - |
| 43 | - | - | - | - | - | - | " | " | " | " | " | " | " | " | " | L-Ala | " | " | " | " | " | " | " | " | - | - | - | - |
| 44 | - | - | - | - | - | - | " | " | " | " | " | " | " | " | " | Gly | D-Ala | " | " | " | " | " | " | " | - | - | - | - |
| 45 | - | - | - | - | - | - | " | " | " | " | " | " | " | " | " | " | " | D-Gln | " | " | " | " | " | " | - | - | - | - |
| 46 | - | - | - | - | - | - | " | " | " | " | " | " | " | " | " | " | " | Gln | D-Ser | " | " | " | " | " | - | - | - | - |
| 47 | - | - | - | - | - | - | " | " | " | " | " | " | " | " | " | " | " | " | Ser | D-Ala | " | " | " | " | - | - | - | - |
| 48 | - | - | - | - | - | - | " | " | " | " | " | " | " | " | " | " | " | " | " | L-Ala | " | " | " | " | - | - | - | - |
| 49 | - | - | - | - | - | - | " | " | " | " | " | " | " | " | " | " | " | " | " | Gly | L-Leu | " | " | " | - | - | - | - |
| 50 | - | - | - | - | - | - | " | " | " | " | " | " | " | " | " | " | " | " | " | " | Leu | D-Ala | " | " | - | - | - | - |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 51 | - | - | - | - | - | - | " | " | " | " | " | " | " | " | " | " | " | " | " | " | " | L-Ala | " | " | - | - | - | - |
| 52 | - | - | - | - | - | - | " | " | " | " | " | " | " | " | " | " | " | " | " | " | " | Gly | D-Cys | " | - | - | - | - |
| 53 | - | - | - | - | - | - | " | " | " | " | " | " | " | " | " | " | " | " | " | " | " | " | Cys | D-Asn | - | - | - | - |
| 54 | - | - | - | - | - | - | D-Cys | " | " | " | " | " | " | " | " | " | " | " | " | " | " | " | " | Asn | Ser | - | - | - |
| 55 | - | - | - | - | - | - | Cys | D-Phe | " | " | " | " | " | " | " | " | " | " | " | " | " | " | " | " | " | - | - | - |
| 56 | - | - | - | - | - | - | " | Phe | D-Ala | " | " | " | " | " | " | " | " | " | " | " | " | " | " | " | " | - | - | - |
| 57 | - | - | - | - | - | - | " | " | L-Ala | " | " | " | " | " | " | " | " | " | " | " | " | " | " | " | " | - | - | - |
| 58 | - | - | - | - | - | - | " | " | Gly | D-Ala | " | " | " | " | " | " | " | " | " | " | " | " | " | " | " | - | - | - |
| 59 | - | - | - | - | - | - | " | " | " | L-Ala | " | " | " | " | " | " | " | " | " | " | " | " | " | " | " | - | - | - |
| 60 | - | - | - | - | - | - | " | " | " | Gly | D-Arg | " | " | " | " | " | " | " | " | " | " | " | " | " | " | - | - | - |
| 61 | - | - | - | - | - | - | " | " | " | " | Arg | D-Ile | " | " | " | " | " | " | " | " | " | " | " | " | " | - | - | - |
| 62 | - | - | - | - | - | - | " | " | " | " | " | D-Met | " | " | " | " | " | " | " | " | " | " | " | " | " | - | - | - |
| 63 | - | - | - | - | - | - | " | " | " | " | " | DaIle | " | " | " | " | " | " | " | " | " | " | " | " | " | - | - | - |
| 64 | - | - | - | - | - | - | " | " | " | " | " | Ile | D-Asp | " | " | " | " | " | " | " | " | " | " | " | " | - | - | - |
| 65 | - | - | - | - | - | - | " | " | " | " | " | " | Asp | D-Arg | " | " | " | " | " | " | " | " | " | " | " | - | - | - |
| 66 | - | - | - | - | - | - | " | " | " | " | " | " | " | Arg | D-Ile | " | " | " | " | " | " | " | " | " | " | - | - | - |
| 67 | - | - | - | - | - | - | " | " | " | " | " | " | " | " | D-Met | " | " | " | " | " | " | " | " | " | " | - | - | - |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 68 | – | – | – | – | – | – | " | " | " | " | " | " | " | " | DaIle | " | " | " | " | " | " | " | " | " | " | – | – | – |
| 69 | – | – | – | – | – | – | " | " | " | " | " | " | " | " | Ile | D-Ala | " | " | " | " | " | " | " | " | " | – | – | – |
| 70 | – | – | – | – | – | – | " | " | " | " | " | " | " | " | " | L-Ala | " | " | " | " | " | " | " | " | " | – | – | – |
| 71 | – | – | – | – | – | – | " | " | " | " | " | " | " | " | " | Gly | D-Ala | " | " | " | " | " | " | " | " | – | – | – |
| 72 | – | – | – | – | – | – | " | " | " | " | " | " | " | " | " | " | Ala | D-Gln | " | " | " | " | " | " | " | – | – | – |
| 73 | – | – | – | – | – | – | " | " | " | " | " | " | " | " | " | " | " | Gln | D-Ser | " | " | " | " | " | " | – | – | – |
| 74 | – | – | – | – | – | – | " | " | " | " | " | " | " | " | " | " | " | " | Ser | D-Ala | " | " | " | " | " | – | – | – |
| 75 | – | – | – | – | – | – | " | " | " | " | " | " | " | " | " | " | " | " | " | L-Ala | " | " | " | " | " | – | – | – |
| 76 | – | – | – | – | – | – | " | " | " | " | " | " | " | " | " | " | " | " | " | Gly | L-Leu | " | " | " | " | – | – | – |
| 77 | – | – | – | – | – | – | " | " | " | " | " | " | " | " | " | " | " | " | " | " | Leu | D-Ala | " | " | " | – | – | – |
| 78 | – | – | – | – | – | – | " | " | " | " | " | " | " | " | " | " | " | " | " | " | " | L-Ala | " | " | " | – | – | – |
| 79 | – | – | – | – | – | – | " | " | " | " | " | " | " | " | " | " | " | " | " | " | " | Gly | D-Cys | " | " | – | – | – |
| 80 | – | – | – | – | – | – | " | " | " | " | " | " | " | " | " | " | " | " | " | " | " | " | Cys-D-Asn | | " | – | – | – |
| 81 | – | – | – | – | – | – | " | " | " | " | " | " | " | " | " | " | " | " | " | " | " | " | " | Asn-D-Ser | | – | – | – |
| 82 | – | – | – | – | – | – | D-Cys | " | " | " | " | " | " | " | " | " | " | " | " | " | " | " | " | " | Ser | Phe | – | – |
| 83 | – | – | – | – | – | – | Cys | D-Phe | " | " | " | " | " | " | " | " | " | " | " | " | " | " | " | " | " | " | – | – |
| 84 | – | – | – | – | – | – | " | Phe | D-Ala | " | " | " | " | " | " | " | " | " | " | " | " | " | " | " | " | " | – | – |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 85 | – | – | – | – | – | – | " | " | L-Ala | " | " | " | " | " | " | " | " | " | " | " | " | " | " | " | " | " | – | – |
| 86 | – | – | – | – | – | – | " | | Gly | D-Ala | " | " | " | " | " | " | " | " | " | " | " | " | " | " | " | " | – | – |
| 87 | – | – | – | – | – | – | " | " | " | L-Ala | " | " | " | " | " | " | " | " | " | " | " | " | " | " | " | " | – | – |
| 88 | – | – | – | – | – | – | " | " | " | Gly | D-Arg | " | " | " | " | " | " | " | " | " | " | " | " | " | " | " | – | – |
| 89 | – | – | – | – | – | – | " | " | " | " | Arg | D-Ile | " | " | " | " | " | " | " | " | " | " | " | " | " | " | – | – |
| 90 | – | – | – | – | – | – | " | " | " | " | " | D-Met | " | " | " | " | " | " | " | " | " | " | " | " | " | " | – | – |
| 91 | – | – | – | – | – | – | " | " | " | " | " | DaIle | " | " | " | " | " | " | " | " | " | " | " | " | " | " | – | – |
| 92 | – | – | – | – | – | – | " | " | " | " | " | Ile | D-Asp | " | " | " | " | " | " | " | " | " | " | " | " | " | – | – |
| 93 | – | – | – | – | – | – | " | " | " | " | " | " | Asp | D-Arg | " | " | " | " | " | " | " | " | " | " | " | " | – | – |
| 94 | – | – | – | – | – | – | " | " | " | " | " | " | " | Arg | D-Ile | " | " | " | " | " | " | " | " | " | " | " | – | – |
| 95 | – | – | – | – | – | – | " | " | " | " | " | " | " | " | D-Met | " | " | " | " | " | " | " | " | " | " | " | – | – |
| 96 | – | – | – | – | – | – | " | " | " | " | " | " | " | " | DaIle | " | " | " | " | " | " | " | " | " | " | " | – | – |
| 97 | – | – | – | – | – | – | " | " | " | " | " | " | " | " | Ile | D-Ala | " | " | " | " | " | " | " | " | " | " | – | – |
| 98 | – | – | – | – | – | – | " | " | " | " | " | " | " | " | " | L-Ala | " | " | " | " | " | " | " | " | " | " | – | – |
| 99 | – | – | – | – | – | – | " | " | " | " | " | " | " | " | " | Gly | D-Ala | " | " | " | " | " | " | " | " | " | – | – |
| 100 | – | – | – | – | – | – | " | " | " | " | " | " | " | " | " | " | Ala | L-Gln | " | " | " | " | " | " | " | " | – | – |
| 101 | – | – | – | – | – | – | " | " | " | " | " | " | " | " | " | " | " | Gln | L-Ser | " | " | " | " | " | " | " | – | – |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 102 | - | - | - | - | - | - | " | " | " | " | " | " | " | " | " | " | " | " | Ser | D-Ala | " | " | " | " | " | " | - | - |
| 103 | - | - | - | - | - | - | " | " | " | " | " | " | " | " | " | " | " | " | " | L-Ala | " | " | " | " | " | " | - | - |
| 104 | - | - | - | - | - | - | " | " | " | " | " | " | " | " | " | " | " | " | " | Gly | L-Leu | " | " | " | " | " | - | - |
| 105 | - | - | - | - | - | - | " | " | " | " | " | " | " | " | " | " | " | " | " | " | Leu | D-Ala | " | " | " | " | - | - |
| 106 | - | - | - | - | - | - | " | " | " | " | " | " | " | " | " | " | " | " | " | " | " | L-Ala | " | " | " | " | - | - |
| 107 | - | - | - | - | - | - | " | " | " | " | " | " | " | " | " | " | " | " | " | " | " | Gly | D-Cys | " | " | " | - | - |
| 108 | - | - | - | - | - | - | " | " | " | " | " | " | " | " | " | " | " | " | " | " | " | " | Cys | D-Asn | " | " | - | - |
| 109 | - | - | - | - | - | - | " | " | " | " | " | " | " | " | " | " | " | " | " | " | " | " | " | Asn | D-Ser | " | - | - |
| 110 | - | - | - | - | - | - | " | " | " | " | " | " | " | " | " | " | " | " | " | " | " | " | " | " | Ser | D-Phe | - | - |
| 111 | - | - | - | - | - | - | D-Cys | " | " | " | " | " | " | " | " | " | " | " | " | " | " | " | " | " | " | " | Arg | - |
| 112 | - | - | - | - | - | - | Cys | D-Phe | " | " | " | " | " | " | " | " | " | " | " | " | " | " | " | " | " | " | " | - |
| 113 | - | - | - | - | - | - | " | Phe | D-Ala | " | " | " | " | " | " | " | " | " | " | " | " | " | " | " | " | " | " | - |
| 114 | - | - | - | - | - | - | " | " | L-Ala | " | " | " | " | " | " | " | " | " | " | " | " | " | " | " | " | " | " | - |
| 115 | - | - | - | - | - | - | " | " | Gly | D-Ala | " | " | " | " | " | " | " | " | " | " | " | " | " | " | " | " | " | - |
| 116 | - | - | - | - | - | - | " | " | " | L-Ala | " | " | " | " | " | " | " | " | " | " | " | " | " | " | " | " | " | - |
| 117 | - | - | - | - | - | - | " | " | " | Gly | D-Arg | " | " | " | " | " | " | " | " | " | " | " | " | " | " | " | " | - |
| 118 | - | - | - | - | - | - | " | " | " | " | Arg | D-Ile | " | " | " | " | " | " | " | " | " | " | " | " | " | " | " | - |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 119 | - | - | - | - | - | - | " | " | " | " | " | D-Met | " | " | " | " | " | " | " | " | " | " | " | " | " | " | " | - |
| 120 | - | - | - | - | - | - | " | " | " | " | " | DaIle | " | " | " | " | " | " | " | " | " | " | " | " | " | " | " | - |
| 121 | - | - | - | - | - | - | " | " | " | " | " | Ile | D-Asp | " | " | " | " | " | " | " | " | " | " | " | " | " | " | - |
| 122 | - | - | - | - | - | - | " | " | " | " | " | " | Asp | D-Arg | " | " | " | " | " | " | " | " | " | " | " | " | " | - |
| 123 | - | - | - | - | - | - | " | " | " | " | " | " | " | Arg | D-Ile | " | " | " | " | " | " | " | " | " | " | " | " | - |
| 124 | - | - | - | - | - | - | " | " | " | " | " | " | " | " | D-Met | " | " | " | " | " | " | " | " | " | " | " | " | - |
| 125 | - | - | - | - | - | - | " | " | " | " | " | " | " | " | DaIle | " | " | " | " | " | " | " | " | " | " | " | " | - |
| 126 | - | - | - | - | - | - | " | " | " | " | " | " | " | " | Ile | D-Ala | " | " | " | " | " | " | " | " | " | " | " | - |
| 127 | - | - | - | - | - | - | " | " | " | " | " | " | " | " | " | L-Ala | " | " | " | " | " | " | " | " | " | " | " | - |
| 128 | - | - | - | - | - | - | " | " | " | " | " | " | " | " | " | Gly | D-Ala | " | " | " | " | " | " | " | " | " | " | - |
| 129 | - | - | - | - | - | - | " | " | " | " | " | " | " | " | " | " | Ala | D-Gln | " | " | " | " | " | " | " | " | " | - |
| 130 | - | - | - | - | - | - | " | " | " | " | " | " | " | " | " | " | " | Gln | D-Ser | " | " | " | " | " | " | " | " | - |
| 131 | - | - | - | - | - | - | " | " | " | " | " | " | " | " | " | " | " | " | Ser | D-Ala | " | " | " | " | " | " | " | - |
| 132 | - | - | - | - | - | - | " | " | " | " | " | " | " | " | " | " | " | " | " | L-Ala | " | " | " | " | " | " | " | - |
| 133 | - | - | - | - | - | - | " | " | " | " | " | " | " | " | " | " | " | " | " | Gly | L-Leu | " | " | " | " | " | " | - |
| 134 | - | - | - | - | - | - | " | " | " | " | " | " | " | " | " | " | " | " | " | " | Leu | D-Ala | " | " | " | " | " | - |
| 135 | - | - | - | - | - | - | " | " | " | " | " | " | " | " | " | " | " | " | " | " | " | L-Ala | " | " | " | " | " | - |
| 136 | - | - | - | - | - | - | " | " | " | " | " | " | " | " | " | " | " | " | " | " | " | Gly | D-Cys | " | " | " | " | - |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 137 | – | – | – | – | – | – | " | " | " | " | " | " | " | " | " | " | " | " | " | " | " | " | Cys | D-Asn | " | " | " | – |
| 138 | – | – | – | – | – | – | " | " | " | " | " | " | " | " | " | " | " | " | " | " | " | " | " | Asn | D-Ser | " | " | – |
| 139 | – | – | – | – | – | – | " | " | " | " | " | " | " | " | " | " | " | " | " | " | " | " | " | " | Ser | D-Phe | " | – |
| 140 | – | – | – | – | – | – | " | " | " | " | " | " | " | " | " | " | " | " | " | " | " | " | " | " | " | Phe | D-Arg | – |
| 141 | – | – | – | – | – | – | D-Cys | " | " | " | " | " | " | " | " | " | " | " | " | " | " | " | " | " | " | " | " | Tyr |
| 142 | – | – | – | – | – | – | Cys | D-Phe | " | " | " | " | " | " | " | " | " | " | " | " | " | " | " | " | " | " | " | " |
| 143 | – | – | – | – | – | – | " | Phe | D-Ala | " | " | " | " | " | " | " | " | " | " | " | " | " | " | " | " | " | " | " |
| 144 | – | – | – | – | – | – | " | " | L-Ala | " | " | " | " | " | " | " | " | " | " | " | " | " | " | " | " | " | " | " |
| 145 | – | – | – | – | – | – | " | " | Gly | D-Ala | " | " | " | " | " | " | " | " | " | " | " | " | " | " | " | " | " | " |
| 146 | – | – | – | – | – | – | " | " | " | L-Ala | " | " | " | " | " | " | " | " | " | " | " | " | " | " | " | " | " | " |
| 147 | – | – | – | – | – | – | " | " | " | Gly | D-Arg | " | " | " | " | " | " | " | " | " | " | " | " | " | " | " | " | " |
| 148 | – | – | – | – | – | – | " | " | " | " | Arg | D-Ile | " | " | " | " | " | " | " | " | " | " | " | " | " | " | " | " |
| 149 | – | – | – | – | – | – | " | " | " | " | " | D-Met | " | " | " | " | " | " | " | " | " | " | " | " | " | " | " | " |
| 150 | – | – | – | – | – | – | " | " | " | " | " | DaIle | " | " | " | " | " | " | " | " | " | " | " | " | " | " | " | " |
| 151 | – | – | – | – | – | – | " | " | " | " | " | Ile | D-Asp | " | " | " | " | " | " | " | " | " | " | " | " | " | " | " |
| 152 | – | – | – | – | – | – | " | " | " | " | " | " | Asp | D-Arg | " | " | " | " | " | " | " | " | " | " | " | " | " | " |
| 153 | – | – | – | – | – | – | " | " | " | " | " | " | " | Arg | D-Ile | " | " | " | " | " | " | " | " | " | " | " | " | " |
| 154 | – | – | – | – | – | – | " | " | " | " | " | " | " | " | D-Met | " | " | " | " | " | " | " | " | " | " | " | " | " |

| | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|---|---|---|---|---|---|---|---|---|---|---|---|
| | Ser | Leu | Arg | Arg | Ser | Ser | Cys | Phe | Gly | Gly | Arg | X | Asp | Arg | X | Gly | Ala | Gln | Ser | Gly | Leu | Gly | Cys | Asn | Ser | Phe | Arg | Tyr |
| 155 | – | – | – | – | – | – | " | " | " | " | " | " | " | " | DaIle | " | " | " | " | " | " | " | " | " | " | " | " | " |
| 156 | – | – | – | – | – | – | " | " | " | " | " | " | " | " | Ile | D-Ala | " | " | " | " | " | " | " | " | " | " | " | " |
| 157 | – | – | – | – | – | – | " | " | " | " | " | " | " | " | " | L-Ala | " | " | " | " | " | " | " | " | " | " | " | " |
| 158 | – | – | – | – | – | – | " | " | " | " | " | " | " | " | " | Gly | D-Ala | " | " | " | " | " | " | " | " | " | " | " |
| 159 | – | – | – | – | – | – | " | " | " | " | " | " | " | " | " | " | Ala | D-Gln | " | " | " | " | " | " | " | " | " | " |
| 160 | – | – | – | – | – | – | " | " | " | " | " | " | " | " | " | " | " | Gln | D-Ser | " | " | " | " | " | " | " | " | " |
| 161 | – | – | – | – | – | – | " | " | " | " | " | " | " | " | " | " | " | " | Ser | D-Ala | " | " | " | " | " | " | " | " |
| 162 | – | – | – | – | – | – | " | " | " | " | " | " | " | " | " | " | " | " | " | L-Ala | " | " | " | " | " | " | " | " |
| 163 | – | – | – | – | – | – | " | " | " | " | " | " | " | " | " | " | " | " | " | Gly | L-Leu | " | " | " | " | " | " | " |
| 164 | – | – | – | – | – | – | " | " | " | " | " | " | " | " | " | " | " | " | " | " | Leu | D-Ala | " | " | " | " | " | " |
| 165 | – | – | – | – | – | – | " | " | " | " | " | " | " | " | " | " | " | " | " | " | " | L-Ala | " | " | " | " | " | " |
| 166 | – | – | – | – | – | – | " | " | " | " | " | " | " | " | " | " | " | " | " | " | " | Gly | D-Cys | " | " | " | " | " |
| 167 | – | – | – | – | – | – | " | " | " | " | " | " | " | " | " | " | " | " | " | " | " | " | Cys | D-Asn | " | " | " | " |
| 168 | – | – | – | – | – | – | " | " | " | " | " | " | " | " | " | " | " | " | " | " | " | " | " | Asn | D-Ser | " | " | " |
| 169 | – | – | – | – | – | – | " | " | " | " | " | " | " | " | " | " | " | " | " | " | " | " | " | " | Ser | D-Phe | " | " |
| 170 | – | – | – | – | – | – | " | " | " | " | " | " | " | " | " | " | " | " | " | " | " | " | " | " | " | Phe | D-Arg | " |
| 171 | – | – | – | – | – | – | " | " | " | " | " | " | " | " | " | " | " | " | " | " | " | " | " | " | " | " | Arg | D-Tyr |

Additional peptides of the present invention are obtained, respectively by adding the following amino acid sequences in the positions indicated to each of peptides 1-171:

| 6 | 7 | 8 | 9 | 10 | 11 |
|---|---|---|---|---|---|
| - | - | - | - | - | L-Ser |
| - | - | - | - | L-Ser | L-Ser |
| - | - | - | L-Arg | L-Ser | L-Ser |
| - | - | L-Arg | L-Arg | L-Ser | L-Ser |
| - | L-Leu | L-Arg | L-Arg | L-Ser | L-Ser |
| L-Ser | L-Leu | L-Arg | L-Arg | L-Ser | L-Ser |

Further peptides of the present invention are obtained, respectively, by adding the following amino acid sequences in the positions indicated to each of sequences 12-28, 12-29, 12-30, 12-31, 12-32 or 12-33, as defined herein, wherein all other optically active amino acids have the L-configuration:

| 6 | 7 | 8 | 9 | 10 | 11 |
|---|---|---|---|---|---|
| - | - | - | - | - | D-Ser |
| - | - | - | - | L-Ser | D-Ser |
| - | - | - | - | D-Ser | L-Ser |
| - | - | - | L-Arg | L-Ser | D-Ser |
| - | - | - | L-Arg | D-Ser | L-Ser |
| - | - | - | D-Arg | L-Ser | L-Ser |
| - | - | L-Arg | L-Arg | L-Ser | D-Ser |
| - | - | L-Arg | L-Arg | D-Ser | L-Ser |
| - | - | L-Arg | D-Arg | L-Ser | L-Ser |
| - | - | D-Arg | L-Arg | L-Ser | L-Ser |
| - | L-Leu | L-Arg | L-Arg | L-Ser | D-Ser |

| 6 | 7 | 8 | 9 | 10 | 11 |
|---|---|---|---|---|---|
| - | L-Leu | L-Arg | L-Arg | D-Ser | L-Ser |
| - | L-Leu | L-Arg | D-Arg | L-Ser | L-Ser |
| - | L-Leu | D-Arg | L-Arg | L-Ser | L-Ser |
| - | D-Leu | L-Arg | L-Arg | L-Ser | L-Ser |
| L-Ser | L-Leu | L-Arg | L-Arg | L-Ser | D-Ser |
| L-Ser | L-Leu | L-Arg | L-Arg | D-Ser | L-Ser |
| L-Ser | L-Leu | L-Arg | D-Arg | L-Ser | L-Ser |
| L-Ser | L-Leu | D-Arg | L-Arg | L-Ser | L-Ser |
| L-Ser | D-Leu | L-Arg | L-Arg | L-Ser | L-Ser |
| D-Ser | L-Leu | L-Arg | L-Arg | L-Ser | L-Ser |

The ANF peptides of the present invention may be prepared from their constituent amino acids by standard methods of protein synthesis, e.g., Schroeder et al., "The Peptides", Vol. I, Academic Press, 1965, or Bodanszky et al., "Peptide Synthesis", Interscience Publishers 1966, or McOmie (ed.), "Protective Groups in Organic Chemistry", Plenum Press 1973, and "The Peptides: Analysis, Synthesis, Biology" 2, Chapter 1 by George Barany and R. B. Merrifield, Academic Press, 1980, New York, the disclosures of which are hereby incorporated by reference.

One therapeutic utility of ANF is in congestive heart failure where standard therapy utilizes potent diuretics in combination with peripheral vasodilating drugs. Atrial natriuretic factor combines both of these actions in one molecule which is produced naturally within the body.

In addition to its utility to treat congestive heart failure, a second major therapeutic utility of ANF is essential hypertension. Standard therapy for hypertension utilizes diuretic and peripheral vasodilating drugs. Atrial natriuretic factor incorporates both of these characteristics. A specific use also may be found in the acute treatment of hypertensive crisis such as malignant hypertension where the powerful vasodilating effect of ANF would be paramount.

In addition to these two very broad categories of therapeutic utility, it is possible that those diseases which are characterized by decreases in renal function may benefit because of the favorable action of ANF on renal hemodynamics, especially enhancement of medullary blood flow.

The peptides of the present invention are useful individually or in combination to treat disorders of electrolyte balance and/or altered vascular resistance in a mammalian species in amount of from about 10 picomoles/kg/min. to about 300 nanomoles/kg/min., preferably from about 100 to about 1000 picomoles/kg/min. The peptides may be administered by intravenous infusion, for example in a suitable physiologically acceptable carrier, e.g., saline or phosphate buffered saline.

The peptides of this invention or their amides, or lower alkyl esters or metal salts or acid addition salts with pharmaceutically acceptable acids are administered to a mammalian species, e.g., rats or mice, systemically, either by intravenous, subcutaneous, or intramuscular injection, or by sublingual or nasal administration, in compositions in conjunction with pharmaceutically acceptable vehicles or carriers. For administration by injection or by the nasal route it is preferred to use the peptides in solution in a sterile aqueous vehicle which may also contain other solutes such as buffers or preservatives as well as sufficient quantities of pharmaceutically acceptable salts or of glucose to make the solution isotonic. In addition, when the above compositions are intended for use as sprays for nasal administration they may also contain small amounts of a pharmaceutically acceptable surface-active agent to ensure rapid absorption of the respective peptide by the nasal mucosa. Examples of such surface-active agents are polysorbate 80 (Tween 80), benzalkonium chloride, bile salts such as sodium glycocholate, dioctyl sodium sulfosuccinate (Aerosol OT), and the like. For sublingual administration it is preferred to formulate the peptides of this invention as rapidly dissolving tablets together with solid excipients or carriers such as lactose. Examples of such excipients or carriers are found in standard pharmaceutical texts, e.g., in Remington's Pharmaceutical Sciences, Mack Publishing Company, Easton, Penn., 1970. Intranasal or sublingual administration may be less precise than intravenous injection but it may be a more convenient form of treatment.

When administration of the peptides of the present invention is desired for the obtention of diuretic, natriuretic, vasorelaxant, hypotensive, or antihypertensive effects such as e.g., in the treatment of hypertension, in particular renovascular hypertension, the dosage to be administered will depend upon such factors as the species, age, weight, sex, and condition of the patient and with the chosen form of administration. Generally, treatment is initiated with small dosages substantially less than the optimum dose of the respective peptide. Thereafter, the dosage is increased by small increments until the optimal effect under the given circumstances is reached. In general, the peptides of this invention are most desirably administered at dosage levels which will give effective concentrations of the respective peptide in the blood of the patient without causing any harmful or deleterious side effects, and preferably at a level that is in the range of from about 0.02 mcg to about 200 mcg per kilogram body weight, although as aforementioned variations will occur. However, for infusion a dosage level that is in the range of from about 0.1 mcg to about 1000 mcg/minute/kg is most desirably employed to achieve effective results. Single doses may be administered in a dosage level of from about 0.01 to about 10 mg in one or more divided doses.

It is often desirable to administer the peptides of this invention continuously over prolonged periods of time in long-acting, slow-release, or depot dosage forms. Such dosage forms may either contain a pharmaceutically acceptable salt of the respective peptide having a low degree of solubility in body fluids, for example one of those salts described above, or they may contain the peptide in the form of a water-soluble salt together with a protective carrier which prevents rapid release. In the latter case, for example, the peptide may be formulated with a non-antigenic partially hydrolyzed gelatin in the form of a viscous liquid; or the peptide may be absorbed on a pharmaceutically acceptable solid carrier, for example zinc hydroxide, and may be administered in suspension in a pharmaceutically acceptable liquid vehicle; or the peptide may be formulated in gels or suspensions with a protective non-antigenic hydrocolloid, for example sodium carboxymethylcellulose, polyvinylpyrrolidone, sodium alginate, gelatin, polygalacturonic acids, for example, pectin, or certain mucopolysaccharides, together with aqueous or nonaqueous pharmaceutically acceptable liquid vehicles, preservatives, or surfactants. Examples of such formulations are found in standard pharmaceutical texts, e.g., in Remington's Pharmaceutical Sciences cited above. Long-acting, slow-release preparations of the peptides of this invention may also be obtained by microencapsulation in a pharmaceutically acceptable coating material, for example gelatin, polyvinyl alcohol or ethyl cellulose. Further examples of coating materials and the processes used for microencapsulation are described by J. A. Herbig in Encyclopedia of Chemical Technology, Vol. 13, 2nd Ed., Wiley, New York 1967, pp. 436-456. Such formulations, as well as suspensions of salts of the peptide which are only sparingly soluble in body fluids, are designed to release from about 0.02 mcg to about 20 mcg of the peptide per kilogram body weight per day, and are preferably administered by intramuscular injection. Alternatively, some of the solid dosage forms listed above, for example certain sparingly water-soluble salts or dispersions in or adsorbates on solid carriers of salts of the peptides, for example dispersions in a neutral hydrogel of a polymer of ethylene glycol methacrylate or similar monomers cross-linked as described in U.S. Pat. No. 3,551,556 may also be formulated in the form of pellets releasing about the same amounts as shown above and may be implanted subcutaneously or intramuscularly.

The following examples illustrate the present invention without, however, limiting the same thereto.

## EXAMPLE 1

The following table shows the potencies of representative peptides of the present invention in relaxing rabbit aorta compared to known C-carboxyl terminal peptides of sequences 6-33 and 8-33 when tested essentially by the method of Winquist et. al., European Journal of Pharmacology 102:169-173 (1984).

| Peptide Sequence | C-Terminal Group | Amino Acid Substitution | Relative Potency |
|---|---|---|---|
| 6-33 | -COOH | | 1 |
| 8-33 | -COOH | | 1 |
| 11-31 | $-CONH_2$ | | 0.32 |
| 11-31 | $-CONH_2$ | $D\text{-}Ser^{11}$ | 0.12 |
| 11-31 | $-CONH_2$ | $D\text{-}Cys^{12}$ | 0.09 |
| 11-31 | $-CONH_2$ | $D\text{-}Phe^{13}$ | 0.008 |
| 11-31 | $-CONH_2$ | $D\text{-}Ala^{22}$ | 0.06 |
| 11-31 | $-CONH_2$ | $D\text{-}Gln^{23}$ | 0.28 |
| 11-31 | $-CONH_2$ | $D\text{-}Ser^{24}$ | 0.04 |
| 10-31 | $-CONH_2$ | | 0.25 |
| 10-31 | $-CONH_2$ | $D\text{-}Ala^{14}$ | 0.22 |
| 10-31 | $-CONH_2$ | $L\text{-}Ala^{14}$ | 0.05 |

| Peptide Sequence | C-Terminal Group | Amino Acid Substitution | Relative Potency |
|---|---|---|---|
| 10-31 | $-CONH_2$ | $D\text{-}Ala^{15}$ | 0.03 |
| 10-31 | $-CONH_2$ | $L\text{-}Ala^{15}$ | 0.26 |
| 10-31 | $-CONH_2$ | $D\text{-}Ile^{17}$ | 0.005 |
| 10-31 | $-CONH_2$ | $D\text{-}Asp^{18}$ | 0.003 |
| 10-31 | $-CONH_2$ | $D\text{-}Arg^{19}$ | 0.005 |
| 10-31 | $-CONH_2$ | $D\text{-}Ala^{25}$ | 0.36 |
| 10-31 | $-CONH_2$ | $D\text{-}Leu^{26}$ | 0.02 |
| 10-31 | $-CONH_2$ | $D\text{-}Ala^{27}$ | 0.25 |
| 8-30 | $-CONH_2$ | | 0.15 |
| 8-31 | $-CONH_2$ | | 0.33 |
| 8-33 | $-CONH_2$ | | 1 |
| 8-33 | -COOH | $D\text{-}Arg^{16}$ | 0.25 |
| 8-33 | -COOH | $D\text{-}Ile^{20}$ | 0.13 |
| 8-33 | -COOH | $D\text{-}Ala^{21}$ | 0.64 |
| 8-33 | -COOH | $L\text{-}Ala^{21}$ | 2.1 |
| 8-33 | -COOH | $L\text{-}Ala^{25}$ | 0.74 |
| 8-33 | -COOH | $L\text{-}Ala^{27}$ | 0.77 |
| 8-33 | -COOH | $D\text{-}Cys^{28}$ | 0.2 |
| 8-33 | -COOH | $D\text{-}Asn^{29}$ | 0.33 |
| 8-33 | -COOH | $D\text{-}Ser^{30}$ | 0.84 |
| 8-33 | -COOH | $D\text{-}Phe^{31}$ | 0.41 |

CLAIMS:

1. A peptide of the amino acid sequence

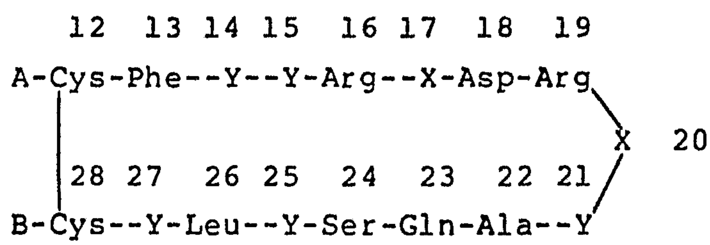

wherein X is L-Ile, D-Ile, D-allo-Ile, L-Met or D-Met, Y is Gly, L-Ala or D-Ala, A is H, Ser, Ser-Ser, Arg-Ser-Ser, Arg-Arg-Ser-Ser, Leu-Arg-Arg-Ser-Ser or Ser-Leu-Arg-Arg-Ser-Ser and B is OH, Asn, Asn-Ser, Asn-Ser-Phe, Asn-Ser-Phe-Arg, or Asn-Ser-Phe-Arg-Tyr, and all optically active amino acid residues may be D or L provided at least one amino acid has D-configuration, and the amides, lower alkyl esters and the physiologically acceptable metal salts and acid addition salts thereof.

2. A peptide of Claim 1 wherein the C-terminal group is -COOH.

3. A peptide of Claim 1 wherein the C-terminal group is $-CONH_2$.

4. A peptide of Claim 1 wherein B is a C-terminal carboxyl group.

5. A peptide of Claim 1 wherein B contains Asn-.

6. A pharmaceutical composition comprising a peptide of Claim 1 in a combination with a pharmaceutically acceptable carrier.

7. The use of a peptide of Claim 1 for the manufacture of a medicament useful for treating a disorder of electrolyte balance.

8. The use of a peptide of Claim 1 for the manufacture of a medicament useful for treating a disorder of altered vacular resistance.

9. The use of a peptide of Claim 1 for the manufacture of a medicament useful for treating essential hypertension.

10. The use of a peptide of Claim 1 for the manufacture of a medicament useful for treating congestive heart failure.